# EUROPEAN PATENT APPLICATION

(11) **EP 3 470 524 A1**
(43) Date of publication of application: **17.04.2019**
(21) Application number: 17196084.2
(22) Date of filing: 12.10.2017
(51) Int. Cl.: C12P 7/06, C12P 7/16, C12P 7/14

(54) **PROCESS FOR THE PRODUCTION OF ALCOHOLS**

(71) Applicant: Technische Universität München, 80333 München (DE)
(72) Inventor: Doll, Kathrin, 86807 Buchloe (DE); Weuster-Botz, Dirk, 80634 München (DE)
(74) Representative: Schiweck Weinzierl Koch Patentanwälte Partnerschaft mbB

(57) **Abstract**

The present invention relates to a process for the production of alcohols by *Clostridium carboxidivorans* in a suitable medium comprising the gases carbon dioxide and carbon monoxide in a first reactor (R1) at a pH value of about 6. In the first step at least one alcohol is formed. Following the cultivation in the first reactor (R1), at least a part of the medium present in reactor (R1), comprising cells of *Clostridium carboxidivorans* and the products formed in the first step, is transferred from reactor (R1) into a second reactor (R2). In the second reactor (R2) *Clostridium carboxidivorans* is cultivated at a pH of at least 5, wherein at least one alcohol is formed.

## Description

### TECHNICAL FIELD OF THE INVENTION

The present invention relates to a process for the production of alcohols by *Clostridium carboxidivorans* in a suitable medium comprising at least one of the gases carbon dioxide or carbon monoxide in a first reactor (R1) at a pH value of about 6. In the first step at least one alcohol is formed. Following the cultivation in the first reactor (R1), at least a part of the medium present in reactor (R1), comprising cells of *Clostridium carboxidivorans* and the products formed in the first step, is transferred from reactor (R1) into a second reactor (R2). In the second reactor (R2) *Clostridium carboxidivorans* is cultivated at a pH of at least 5, wherein at least one alcohol is formed.

### BACKGROUND ART

The limited amount of fossil resources available and the increasing demand thereof encourages the exploration of alternative processes for the production of basic chemicals and fuels. Sustainable processes applying microorganisms could represent possible solutions.

Gas fermentation is a relatively new field in the cultivation of bacteria. Its application is limited by two aspects. The gases hydrogen, carbon monoxide and carbon dioxide represent as autotrophic sources, in contrast to heterotrophic carbon sources, a significantly lower yielding energy source. Due to this difference, the metabolism of acetogenic bacteria is characterized by slow growth (Mohammadi, M. et al., 2011, Bioconversation of synthesis gas to second generation biofuels: A review, Renewable and Sustainable Energy Reviews 15 (9), 4255 to 4273.). On the other hand, the subtrates, in particular carbon monoxide and hydrogen, exibit in comparison with oxygen only low solubilities, which results in mass transfer limitations.

*Clostridium carboxidivorans* is of interest in this respect since it is one of the few acetogenic bacteria which are able to produce longer chained carbon compounds, like butanoic acid and butanol directly from synthesis gas as well as hexanoixc acid and hexanol, besides acetic acid and ethanol which are usually made by acetogenic bacteria (Ukpong, M. N. et al., 2012, Physiological response of Clostridium carboxydivorans during conversion of synthesis gas to solvents in a gas-fed bioreactor, Biotechnol. Bioeng. 109 (11)).

Ramachandriya et al. (Ramachandriya, KD. et al. 2013, Carbon dioxide conversion to fuels and chemicals using a hybrid green process, Applied Energy 112, 289 to 299) disclose the production of hexanol and hexanoic acid during the cultivation of *Clostridium carboxidivorans.* These experiments were conducted mixotrophic with corn stip liquor and cotton seed extract containing medium. The flask cultures were daily gased with a hydrogen/carbon dioxid gas composition. 0.7 g L⁻¹ hexanol und 0.42 g L⁻¹ hexanoic acid were obtained.

Phillips et al. (Phillips, J. R. et al., 2015, Butanol and hexanol production in Clostridium carboxidivans syngas fermentation: Medium development and culture techniques, Bioresource Technology, 190,114 to 121) report the production of hexanol by *Clostridium carboxidivans* using a medium free of complex ingredients. A hexanol concentration up to 0.94 g L⁻¹ could be reached by adapted experimental conditions and a respective medium in anaerobic flasks. A cultivation time of 360 h was necessary.

A further group (Ramio-Pujol, S. et al. (2015 b): Incubation at 25 °C prevents acid crash and enhances alcohol production in Clostridium carboxidivans P7, Bioresource Technology, 192, 296 to 303) describes the increased production of alcohols at reduced temperature. Batch cultivations in regularly gased anaerobic flasks at reduced temperature of 25 °C reduced the metabolic rates, but yielded up to 8.21 mM (0.84 g L⁻¹) hexanol and 9.2 mM (1.05 g L⁻¹) hexanoic acid, while at the optimum cultivation temperature the alcohol formation ceased due to the so called "acid crash". "Acid crash" is a term describing the lack of the metabolic change from acid formation to alcohol production due to a too quck acid accumulation and bacteria growth.

Datar et al. (Datar, R. P. et al., 2004, Fermentation of biomass-generated producer gas to ethanol, Biotechnol. Bioeng. 86 (5), 587 to 594) disclose a continuous cultivation of *Clostridium carboxidivans,* comprising four phases. 1) Growth in batch process with continuous gas feed; 2) continuous processing at 1.5 mLmin⁻¹ (operating volume 4 L) with parallel refill of medium nutrients; 3) switching from bottled clean gas to synthesis gas from biomass gasification of switchgrass at constant biomass concentration; 4) operation of the liquid phase in a batch process for observation of the further growth, whereby it has been switched again to clean bottled synthesis gas.

Ahmed et al. (Ahmed, A. et al., 2006, Effects of biomass-generated producer gas constituents on cell growth, product distribution and hydrogenase activity of Clostridium carboxidivans P7T, Biomass and Bioenergy, 30 (7), 665 to 672) disclose a continuous cultivation of *Clostridium carboxidivans* in a stirred tank reactor, mentioning just acetic acid and ethanol as products. The conducted experiments likewise comprise four phases: 1) batch process with pH-control between 5.85 and 5.25, 2) continuous feed of 0.36 mL min⁻¹ (operating volume 3 L) with one-sided pH-control for pH 5.25; 3) switch from synthetic gas to synthesis gas generated by biomass gasification of switchgrass with control for pH 5.35 (filtered with a 0.025 µm filter); 4) Exchange of the filter by a 0.2 µm filter with pH-rise).

Fernandez-Naveira et al. (Fernandez-Naveira, A. et al. 2016, Efficient butanol-ethanol (B-E) production from carbon monoxide fermentation by Clostridium carboxidivans, Applied microbiology and biotechnology, 100 (7), 3361 to 3370) report autotrophic cultivations of *Clostridium carboxidivans* in a stirred tank reactor, in which by a pH switch or a partial exchange of the medium high ethanol and butanol concentrations (5.55 g L⁻¹ and 2.66 g L⁻¹) could be achieved.

US 8,592,191 discloses a process for the fermentation of synthesis gas comprising the cultivation of an acetogenic bacterium, for example *Clostridium carboxidivans,* a first cultivation in a pre-reactor and a cultivation in a second main reactor. Wherein the first culture has a pH-value of 6.5 or less. The transfer of the first culture into the second culture is dependent of the cell density.The process is directed for example to the production of ethanol or butanol.

However, a process for the production of alcohols with *Clostridium carboxidivans,* which combines growth with improved product formation has not been disclosed.

Thus, the aim of the present invention is to create a further process for the production of alcohols wth *Clostridium carboxidivans.*

### SUMMARY OF THE INVENTION

The invention relates to a process for the production of alcohols, comprising the steps:
a) Cultivating *Clostridium carboxidivorans* in a suitable medium comprising the gases carbon dioxide and carbon monoxide in a first reactor (R1) at a pH value of about 6, wherein at least a part of the least one gas is converted into at least one alcohol.
b) Transferring at least a part of the medium present in reactor (R1), comprising cells of *Clostridium carboxidivorans* and the products formed in step a), from reactor (R1) into a second reactor (R2).
c) Cultivating *Clostridium carboxidivorans,* in reactor (R2) at a pH value of about 5, wherein at least one alcohol is formed.

The process of the present invention allows combining conditions suitable for promoting growth of biomass of *Clostridium carboxidivorans* in step a) with conditions which promote conversion of organic acids formed under the growth conditions in step a) to alcohols in step b) and step c). Due to these process conditions the overall yield of alcohols in the process can be increased without sacrificing growth of biomass.

Furthermore, the conditions in step c) significantly promote the formation of longer chain alcohols, for example butanol and hexanol, which are formed in a comparatively small amount or not at all during the cultivation under the "growth conditions" in step a).

The combination of biomass growth promoting conditions and conditions which promote the conversion of organic acids into alcohols and the formation of longer chain alcohols in one process is of particular advantage, if the process is conducted as continuous process.

### BRIEF DESCRIPTION OF THE FIGURES

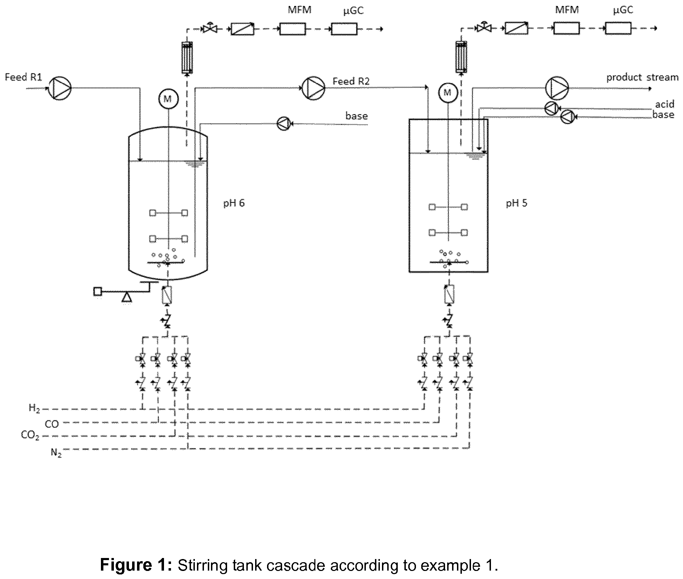

**Fig. 1** depicts a scheme of the Stirring tank cascade according to example 1.
**Fig. 2** depicts a continuous cultivation of C. *carboxidivans* in a stirring tank cascade comprising two stirring tanks according to example 1 with D_{R1} = 0.12 h⁻¹ ; D_{R2} = 0.08 h⁻¹. Hurst Medium; 1200 rpm, 10 mL 4 % Cys-HCI, Gas mixture CO: CO₂ = 60: 40, 37 °C, 1.01 bar; V_{R1} = 1.0 L; V_{R2} = 1.5 L.
**Fig.** 3 depicts the cumulative amount of the products of the stirring tank cascade with *C*. *carboxidivorans* according to example 1 with D_{R1} = 0.12 h⁻¹ ; D_{R2} = 0.08 h⁻¹ Hurst Medium; 1200 rpm, 10 mL 4 % Cys-HCI, Gasmischung CO: CO₂ = 60: 40, 37 °C, 1.01 bar; V_{R1} = 1.0 L; V_{R2} = 1.5 L.
**Fig. 4** depicts Equilibrium concentrations in a continuous stirring tank cascades with *C*. *carboxidivorans* at different dilution rates. Hurst Medium; 1200 rpm, 10 mL 4 % Cys-HCI, gas mixture CO: CO₂= 60: 40, 37 °C, 1.01 bar.

### DETAILED DESCRIPTION OF THE INVENTION

The solution of the present invention is described in the following, exemplified in the appended examples, illustrated in the Figures and reflected in the claims.

The present invention provides a process for the production of alcohols.

The process comprises the steps a), b) and c).

In step a) *Clostridium carboxidivorans* is cultivated in a suitable medium.

The medium is preferably a liquid medium, which promotes growth and/or survival of *Clostridium carboxidivorans.* Any liquid medium recognized by the person skilled in the art as suitable for this purpose may be applied. Preferably, the medium comprises yeast extract. Preferably the medium comprises a reduction agent, as specified below.

For example the medium disclosed by Hurst et al. (Hurst K. M., Lewis R. S., 2010, Carbon monoxide partial pressure effects on the metabolic process of syngas fermentation, Biochemical Engineering Journal, 48, 159 to 165) or suitable medium for clostridia cultivation like medium ATCC 1754 or similar may be applied.

Preferably, the medium is sterilized before use, for example by autoclaving.

In step a) the medium comprises the gases carbon dioxide and carbon monoxide. Preferably, the at least one gas is fed directly into the medium by a suitable device like a sparger. Preferably the medium is gassed continuously.

A gas comprising carbon dioxide and carbon monoxide may be fed into the medium. Preferably, if the gas fed into the medium comprises carbon dioxide and carbon monoxide, the ratio between carbon dioxide, based on volume-%, is 9 to 0.1, more preferably 4 to 0.6, most preferably 2.5 to 0.8, particularly preferred 1.5. The gas fed into the medium may comprise further gases, like intert gases, for example nitrogen.

Preferably, the medium is purged with the at least one gas until the medium is essentially free of oxygen.

The pressure in the first reactor (R1) in step a) may be 1 to 20 bar, preferably 1 to 10 bar, more preferably 1 to 5 bar, most preferably 1 to 1.5 bar.

Preferably the gas flow in step a) is in vvm 0.02 to 0.2 vvm, more preferably 0.05 to 0.16 vvm , most preferably 0.1 vvm

Further, a reduction agent may be present in step a).

The reduction agent may be selected from cysteine, or a salt thereof, sodium thioglycolate, sodium sulfide nonahydrate, iron sulfide, dithiothreitol, sodium dithionite and/or hydrogen gas. Preferably, the reduction agent is selected from cysteine or a salt thereof or hydrogen, more preferably cysteine or a salt thereof.

If the reduction agent is selected from cystein or a salt thereof, the concentration in the medium of reactor (R1) in step a) may be 0.1 to 1g·l⁻¹, preferably 0.15 to 0.9 g·l⁻¹, more preferably 0.3 to 0.6 g·l⁻¹.

The reduction agent may be added in one portion at the beginning of step a) or stepwise during step a). If hydrogen is present as a reduction agent, it may be fed as gas mixture together with carbon dioxide and/or carbon monoxide, preferably directly into the medium.

Furthermore, in step a) an antifoaming agent may be present. The antifoaming agent may be selected from polypropylenglycole.

The cultivation in step a) is conducted at a pH value of about 6.

The term "about" means in this respect, that the pH value may be slightly higher or lower than 6. "About" further means that the pH value may be 5.8 to 6.2, preferably, 5.85 to 6.15, more preferably 5.9 to 6.1.

During the cultivation in step a), the pH-value may be monitored and maintained at a pH of about 6, by the addition of at least one suitable acid and/or at least one suitable base in order to compensate changes of the pH value. The monitoring of the pH-value may be performed by conventional means according to the state of the art.

The acid may be an aqueous solution of H₂SO₄ in a suitable concentration.

The base may be an aqueous solution of NaOH in a suitable concentration.

The dilution rate in step a) is preferably 0.01 to 0.2 h⁻¹, more preferably 0.04 to 0.16 h⁻¹, most preferably 0.09 to 0.14 h⁻¹ and in particularly preferred 0.10 to 0.13 h⁻¹.

Preferably in reactor (R1), during steady state, a biomass of 1 to 6 g/l, more preferably 1.2 to 4 g/l, most preferably 1.4 to 2 g/l and particularly preferred 1.5 to 1.8 g/l is present.

Preferably, the residence time in step a) is 2 to 20 h, more preferably 5 to 12 h and most preferably 7 to 10 h.

The temperature in step a) is preferably 20 to 40 °C, more preferably 25 to 38 °C, most preferably 30 to 39 °C and particularly preferred 35 to 38 °C.

The reactor (R1) in step a) may be a stirred tank reactor, bubble column or gas-lift reactor. Preferably, the reactor (R1) is a stirred tank reactor.

The medium in reactor (R1) in step a) may be stirred mechanically by a suitable device.

If the medium in the reactor (R1) in step a) is stirred, the specific stirring power (PV) is preferably, 2 to 20 W/l, more preferably 6 to 15 W/l, most preferably 8 to 13 W/l.

During step a) at least one alcohol is formed.

Preferably, the at least one alcohol is an aliphatic alcohol.

The at least one alcohol is preferably a monoalcohol.

The at least one alcohol preferably comprises 1 to 10 carbon atoms.

Preferably, the at least one alcohol formed in step a) is selected from ethanol, butanol and/or hexanol.

Besides the at least one alcohole, other molecules may be formed. In particular, the corresponding organic acid or the corresponding salts therof of the at least one alcohol may be formed.

The corresponding organic acid or the corresponding salt preferably comprises 1 to 10 carbon atoms.

Thes organic acid may be selected for example from acitic acid or acetate, butanoic acid or butyrate, hexanoic acid or hexanoate.

In step b) at least a part of the medium present in reactor (R1) is transferred into a second reactor (R2). The medium may be completely transferred in one step if the process is conducted as a batch process or continuously, if the process is conducted as continuous process. The transferred medium comprises cells of *Clostridium carboxidivorans* and products formed in step a).

Preferably, if the process is conducted as continuous process, the volume flow into reactor (R1) is equal to the volume flow leaving reactor (R1) by transfer to reactor (R2).

Preferably, the amount of organic acids or the corresponding salts thereof in the medium of step b), based on the overall weight, is higher than in the medium after finishing step c).

In step c) *Clostridium carboxidivans* is cultivated at a pH value of about 5.

The term "about" means in this respect, that the pH value may be slightly higher or lower than 5. "About" means in this respect that the pH value may be 4.8 to 5.2, preferably, 4.85 to 5.15, more preferably 4.9 to 5.1.

During the cultivation in step a), the pH-value may be monitored and maintained at a pH of about 6, by the addition of at least one suitable acid and/or at least one suitable base in order to compensate changes of the pH value. The monitoring of the pH-value may be performed by conventional means according to the state of the art.

The acid may be an aqueous solution of H₂SO₄ in a suitable concentration.

The base may be an aqueous solution of NaOH in a suitable concentration.

In step c), preferably the cultivation is carried out in the medium transferred in step b). Preferably, the medium in step c) comprises products formed in step a).

Preferably, in step c) at least one of the gases carbon monoxide or carbon monoxide is present. Preferably, the at least one gas is fed directly into the medium by a suitable device.

A gas comprising carbon dioxide and carbon monoxide may be fed into the medium in step c). Preferably, if the gas fed into the medium comprises carbon dioxide and carbon monoxide, the ratio between carbon dioxide, based on volume-%, is 9 to 0.1, more preferably 4 to 0.6, most preferably 2.5 to 0.8, particularly preferred 1.5. The gas fed into the medium may comprise further gases, like intert gases, for example nitrogen.

Preferably, the medium is purged with the at least one gas until the medium is essentially free of oxygen.

The initial pressure in step c) is preferably 1 to 20 bar, more preferably 1 to 10 bar, most preferably 1 to 5 bar, and particularly preferred 1 to 1.5 bar.

The gas flow in step c) is preferably 0.01 to 0.1vvm , more preferably 0.03 to 0.08vvm , most preferably 0.05 vvm.

Further, a reduction agent may be present in step c).

The reduction agent may be selected from cystein or a salt thereof, sodium thioglycolate, sodium sulfide nonahydrate, iron sulfide, dithiothreitol, sodium dithionite and/or hydrogen gas.

If the reduction agent is selected from cystein or a salt thereof, the concentration in the medium of reactor (R2) in step c) may be 0.1 to 1g·l⁻¹, preferably 0.15 to 0.9 g·l⁻¹, more preferably 0.3 to 0.6 g·l⁻¹.

The reduction agent may be added in one portion at the beginning of step c) or stepwise during step c). If hydrogen is present as a reduction agent, it may be fed as gas mixture together with carbon dioxide and/or carbon monoxide, preferably directly into the medium.

Furthermore, in step c) an antifoaming agent may be present. The antifoaming agent may be selected from polypropylenglycole.

The residence time in step c) is preferably 2 to 20 h, more preferably 5 to 15 h, most preferably 10 to 13 h.

The temperature in step c) is preferably 20 to 40 °C, more preferably 25 to 38 °C, most preferably 30 to 39 °C and particularly preferred 35 to 38 °C.

The reactor (R2) in step c) may be a stirred tank reactor, bubble column or gas-lift reactor.

The medium in reactor (R2) in step c) may be stirred mechanically by a suitable device.

If the medium in the reactor (R2) in step c) is stirred, the specific stirring power (PV) is preferably, 2 to 20 W/L, more preferably 6 to 18 W/L, most preferably 10 to 16 W/L.

During step c) at least one alcohol is formed.

Preferably, the at least one alcohol is an aliphatic alcohol.

The at least one alcohol is preferably a monoalcohol.

The at least one alcohol preferably comprises 1 to 10 carbon atoms.

Preferably, the at least one alcohol formed in step a) is selected from ethanol, butanol and/or hexanol.

The process may be carried out in a stepwise or continuous manner.

Carrying out the process in stepwise manner may mean that step a) and c) are carried out as a batch process.

If the process is carried out as continuous process, preferably the continuous phase is started with step a) after performing step a) as batch process for at least 5 h more preferably for 5 to 15 h, most preferably for 7 to 12 h, particulary preferred for 10 h.

Preferably the dilution rate in step c) is 0.01 to 0.15 h⁻¹, more preferably 0.04 to 0.12 h⁻¹, most preferably 0.07 to 0.10 h⁻¹..

A better understanding of the present invention and of its advantages will be had from the following examples, offered for illustrative purposes only. The examples are not intended to limit the scope of the present invention in any way.

### EXAMPLES OF THE INVENTION

### Example 1

### Microorganism

The microorganism *Clostridium carboxidivorans* (DSM 15243) was provided by the German Collection of Microorganisms and Cell Cultures (DSMZ, Braunschweig, Germany).

### Continuous cultivation in reactor cascade

For the cultivation with C. *carboxidivorans* a modified medium from Hurst and Lewis (Hurst and Lewis, 2010) was used for the cultivation studies. 0.4 g L⁻¹ Cystein-HCI was used as reducing agent. (see supplementary material)

Pre-culture for cascade cultivations was grown heterotrophically with 5 g L⁻¹ glucose in in 50 mL Hurst and Lewis medium in anaerobic flasks sealed with butyl rubber septums at 37 °C. Therefore 2.5 mL of frozen cell stocks were used. For pH-buffering during growth 15 g L⁻¹ MES (N-morpholinoethansulfonic acid) was used. Exponential growing cells were harvested by centrifugation for 10 minutes at 4500 rpm (Hettich Zentrifuge, Rotixa 50 RS) and resuspended in PBS (phosphaste buffered saline) for inoculation.

Continuous cultivation with *C*. *carboxidivorans* with continuous gas supply in a two-stage reactor cascade was carried out in a 2 L stirred-tank reactor with a working volume of 1 L (Labfors 4, Infors HT, Bottmingen, Switzerland) and a 2.4 L stirred tank reactor with a working volume of 1.5 L (KLF2000, Bioengineering AG, Wald, Switzerland). Complex medium according to Hurst and Lewis was used. Media was autoclaved in anaerobic flaks with butyl rubber septums at 121 °C for 20 min before it was transferred with a peristaltic pump (Watson Marlow, Cornwall, England) to the previously sterilized reactors and anaerobised with the target gas composition of CO: CO₂ = 60 %: 40 % at a flow rate of 5 L h⁻¹ for at least 12 h by mass flow controllers (WMR, Westphal, Mess- und Regeltechnik; Wagner Mess- und Regeltechnik, Offenbach, Germany). Stirred speed was held at 1200 rpm for each reactor (11.8 W L⁻¹ for reactor 1, Labfors 4; 15.1 W L⁻¹ for reactor 2, KLF2000). Cultivation temperature was set to 37 C. pH was adjusted to pH 6.0 in reactor 1 for optimal growth conditions and to pH 5.0 in reactor 2 for favoring solvent formation. Regulation of pH during fermentation was performed with 6M NaOH respectively 2M H₂SO₄ with a unidirectional pH regulation in reactor 1 with base and a bidirectional pH regulation with a dead band of 0.1 in reactor 2 with acid and base. Depending on foam formation 0.1 mL of a 1:10 PPG (poly propylene glycol) solution with Mₙ2000 from Sigma-Aldrich was added manually.

After inoculation of reactor 1, the process was first performed in batch mode for 10 h for guaranteeing of growth of the cells. After that, continuous process mode for liquid phase with fresh anaerobic medium was started with a peristaltic pump (Ismatec, Cole Parmer, Wertheim, Germany) with gas tight tubes (ID = 0.89 mm, Cole Parmer, Wertheim, Germany) and an applied flow rate of D = 0.12 h⁻¹ for reactor 1. For maintenance of liquid stream the feed vessel was equipped with an inflatable flexible nitrogen reservoir (Plastigas, Linde, Germany). Simultaneously, outflow of liquid stream from reactor 1 to reactor 2 was started by peristaltic pump (Infors HT, Bottmingen, Switzerland) and used for gravimetrical regulation of volume constancy in reactor 1. Influx of feed stream for reactor 2 therefore contained cells and products from reactor 1. Volume in reactor 2 was held constant by surface suction with a peristaltic pump. Working volume in reactor 2 of 1.5 L was achieved during feed of fermentation broth from reactor 1 to the supplied 1 L medium in reactor 2.

### Analytical Methods

Samples were taken from the cultivations frequently by sterile single-use syringes for biomass and product concentrations. Optical density (OD) was measured at 600 nm using an UV-vis spectrophotometer (Genesys 10S UV-Vis, Thermo Scientific, Neuss, Germany). For biomass concentration an experimental determined correlation factor was used for estimation of cell dry weight (CDW). Products were quantified by high performance liquid chromatography (HPLC). Detection was performed by refractive index (RI) with an Aminex-HPX-87H ion exchange column as stationary phase and 5 mM H₂SO₄ at a constant flow rate of 0.6 mL min⁻¹ as mobile phase at 40 °C. For quantification of hexanol samples were concentrated with ethyl acetate prior to measurement.

### Exhaust gas analysis

Exhaust gas analysis was analyzed online with a mass flow meter (MFM) (Wagner Mess- und Regeltechnik GmbH, Offenbach, Germany) in combination with a µ-gas chromatograph (µCG 490, Agilent Technologies, Waldbronn, Germany). Having passed the MFM the µGC automatically took a sample every ten minutes for measuring gas composition. For separation of the gases a 1 m COX HI BF column was used at 80 °C with nitrogen as carrier gas. Exhaust gas was cooled with a reflux condenser to 2 °C to minimize evaporation.

**Tabelle 1: Reactor parameters**

| | **Reactor(R1)** | **Reactor(R2)** |
|---|---|---|
| **Operation volume** | 1.0 | 1'.5 |
| **D, h⁻¹** | 0.12 | 0.08 |
| **τ, h** | 8.3 | 12.5 |
| **pH** | 6.0 | 5.0 |
| | | |
| **T, °C** | 37.0 | 37.0 |
| **P/V, WL⁻¹** | 11.8 (1200 rpm) | 15.1 (1200 rpm) |
| **Pᵢₙᵢ, bar abs** | 1.0 | 1.0 |
| **Gas Flow, L h⁻¹** | 5.0 | 5.0 |
| **CO:CO₂, % : %** | 60:40 | 60:40 |

### Results of Example 1

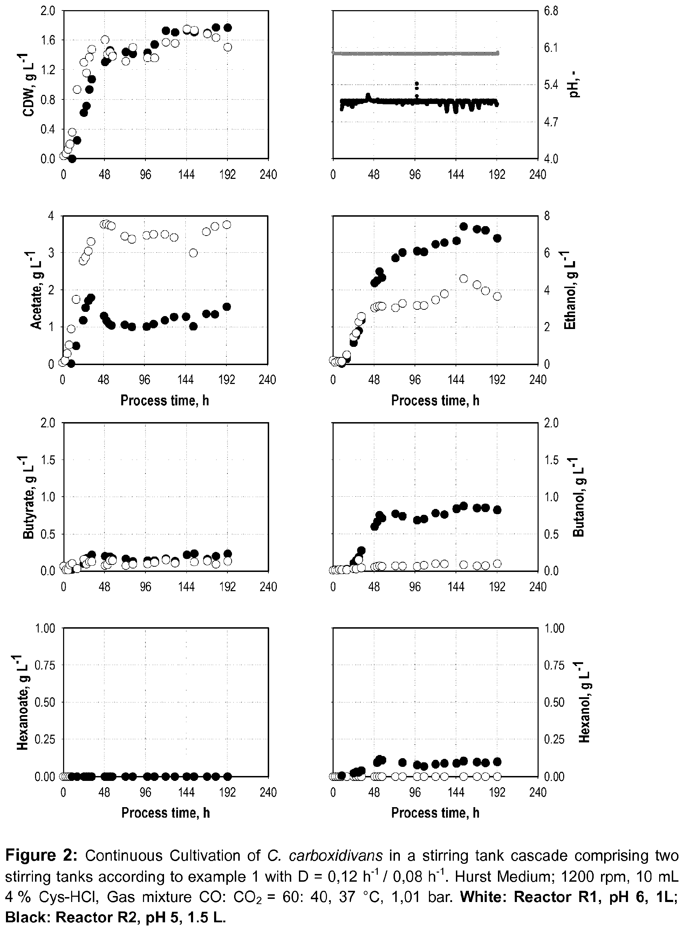

As shown in figure 2 (BTM), in the process of example 1 in steady state 16 gL⁻¹ biomass are produced. The biomass has been the same in both reactors. Thus, growth took place under the conditions of step a) in reactor R1, while nearly no further growth of biomass could be observed for the conditions of step b) in reactor R2.

As further displayed in figure 2, in reactor (R1) about 3.7 gL⁻¹ acetate are produced in equilibrium. With some delay, the formation of 3.5 gL⁻¹ ethanol in equilibrium started. Cells and products from reactor (R1) are transferred to reactor (R2) with the feed.

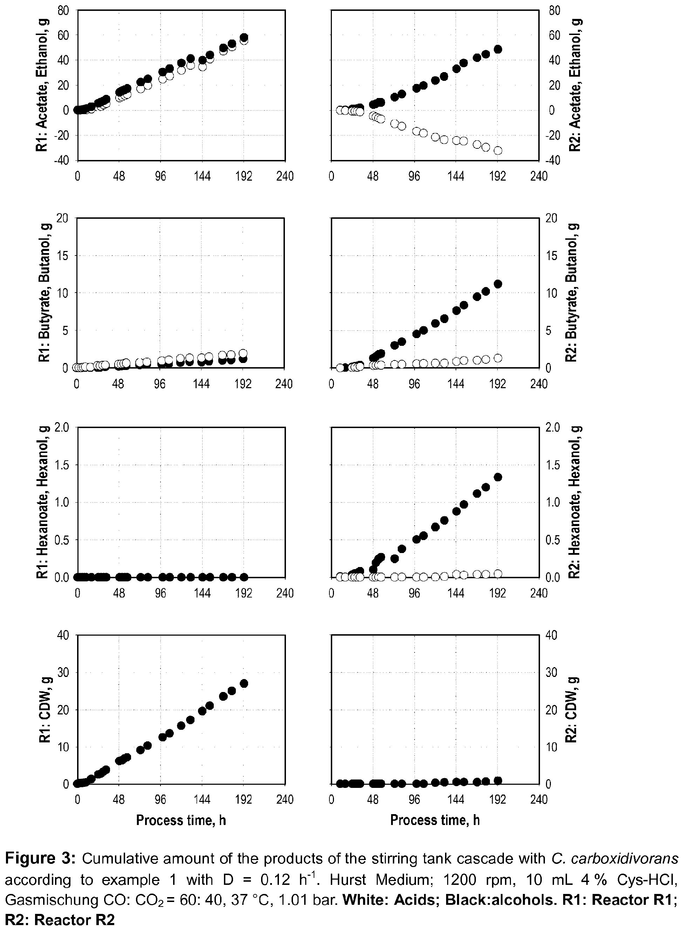

Due to the low pH of about 5, in reactor (R2) the most part of the acetate could be converted to ethanol. The equilibrium concentration of ethanol is 6.5 gL⁻¹. A rest concentration of 1.3 gL⁻¹ in equillibrium of acetic acid remains in reactor (R2). Figure 3 shows the over amounts of the formed products in reactor (R1) and (R2) over time.
Taking the data from figure 2 and figure 3 into account, reactor (R2) serves mainly for converting actetate into ethanol. However, since the amount of formed ethanol in reactor (R2) is higher than the converted acetate, ethanol is also formed *de novo* in reactor (R2).

In contrast to acetate, the formation of only low amounts of butyrate and hexanoat has been observed in reactor (R1) (figure 2).

Of particular technical advantage is the promotion of the formation of longer chain alcohols like butanol (0.8 gL⁻¹ in equilibrium) and hexanol (0.09 gL⁻¹ in equilibrium) by the lower pH value in reactor (R2). It is surpring in this respect, that the amounts of these alcohols formed in step c) in reactor (R2) in comparison to the amounts of butyrate and hexanoat transferred from reactor (R1), show that under the conditions of reactor (R2) hexanol and butanol are produced de *novo.*

**Table 2: Product formation rates in a stirred tank cascade consisting of two reactors at a dilution rate of D = 0.12 h⁻¹. Hurst Medium; 1200 rpm, gas mixture CO : CO₂ = 60 : 40, 37 °C, 1.01 bar. pH 6.0 in reactor (R1); pH 5,0 in reactor (R2).**

| | **Reactor (R1), pH 6** | **Reactor (R2), pH 5** |
|---|---|---|
| Q_{BTM}¹**, g L⁻¹d⁻¹** | 3.46 | 0.18 |
| **Q**_{EtOH}**, g L⁻¹d⁻¹** | 7.34 | 4.54 |
| **Q**_{acetate}**, g L⁻¹d⁻¹** | 7.29 | -4.52 |
| **Q**_{BuOH}**, g L⁻¹d⁻¹** | 0.15 | 1.63 |
| **Q**_{butyrate}**, g L⁻¹d⁻¹** | 0.23 | 0.15 |
| **Q**_{HxOH}**, g L⁻¹d⁻¹** | - | 0.21 |
| **Q**ₕₑₓₐₙₒₐₜₑ**, g L⁻¹d⁻¹** | - | 0.01 |

**Table 3: Product formation in the stirred tank cascade until end of cultivation after 192 h**

| | **Cascade R1 pH 6** | **Cascade R2 pH 5** | **Cascade overall R1+R2** |
|---|---|---|---|
| **M_{CDW}¹, g** | 27.2 | 0.88 | 27.9 |
| **m_{EtOH}, g** | 55.5 | 48.57 | 103.96 |
| **M_{acetate} g** | 58.2 | -32.48 | 25.59 |
| **m_{BuOH}, g** | 1.20 | 11.18 | 12.36 |
| **m_{Butyrate}, g** | 1.93 | 1.29 | 3.20 |
| **m_{HXOH,} g** | **-** | 1.33 | 1.33 |
| **Mₕₑₓₐₙₒₐₜₑ, g** | **-** | 0.05 | 0.05 |

| | | | |
|---|---|---|---|
| ¹ cell dry weight | | | |

### Example 2

Example 2 has been conducted as example 1 but the dilution rate has been lowered from 0.12 h⁻¹/0,.08 h⁻¹ to D = 0.06 h⁻¹/0.04 h⁻¹. Thus, the hydraulic residence times increased.

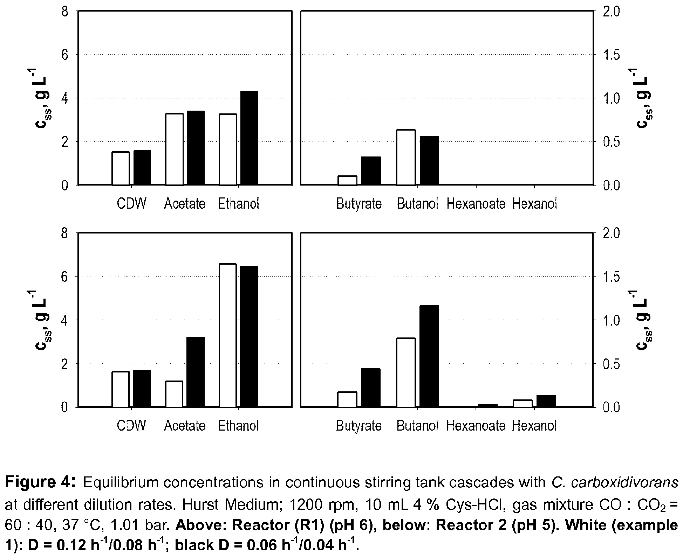

As shown in figure 4, this measure resulted in the formation of an increased amount of ethanol in reactor (R1) and the alcohols butanol and hexanol in reactor (R2).

## Claims

1. Process for the production of alcohols, comprising the steps:
a) cultivating *Clostridium carboxidivorans* in a suitable medium comprising the gases carbon dioxide and carbon monoxide in a first reactor (R1) at a pH value of about 6, wherein at least a part of the least one gas is converted into at least one alcohol,
b) transferring at least a part of the medium present in reactor (R1),comprising cells of *Clostridium carboxidivorans* and the products formed in step a), from reactor (R1) into a second reactor (R2);
c) cultivating *Clostridium carboxidivorans* in reactor (R2) at a pH value of about 5, wherein at least one alcohol is formed, wherein carbon dioxide and carbon monoxide are present.

2. The process according to claim 1, wherein the process is conducted as a continuous process and wherein optionally
I) performing the process as continuous process, is started with step a) after performing step a) as batch process for at least 5 h, preferably for 5 to 15 h, more preferably for 7 to 12 h, most preferably for 10 h and/or
II) the dilution rate in step a) is 0.01 to 0.2 h⁻¹ , preferably 0.04 to 0.16 h⁻¹, more preferably 0.09 to 0.14 h⁻¹, most preferably 0.10 to 0.13 h⁻¹ and/or
III) in step a) in reactor (R1), during steady state, a biomass of 1 to 6 g/l, preferably 1.2 to 4 g/l, more preferably 1.4 to 2 g/l, most preferably 1.5 to 1.8 g/l is present and/or
IV) in reactor (R2), during steady state, in step c) a biomass of 1 to 6 g/l, preferably 1.2 to 4 g/l, more preferably 1.4 to 2 g/l, most preferably 1.5 to 1.8 g/l is present and/or
V) the dilution rate in step c) is 0.01 to 0.15 h⁻¹, preferably 0.04 to 0.12 h⁻¹, more preferably 0.07 to 0.10 h⁻¹.

3. The process according to claim 1 or 2, wherein
I) in step a) a gas comprising carbon monoxide and carbon dioxide is fed into the medium, wherein preferably the ratio of carbon dioxide to carbon dioxide, based on volume-% is 9 to 0.1, more preferably 4 to 0.6, most preferably 2.5 to 0.8, particularly preferred 1.5 and/or
II) the first reactor (R1) is a stirred tank reactor and/or
III) a reduction agent is present in step a) and/or
IV) a reduction agent is present in the step a) and selected from the group consisting of cysteine or a salt thereof, sodium thioglycolate, sodium sulfide nonahydrate, iron sulfide, dithiothreitol, sodium dithionite and hydrogen gas and/or
V) the residence time in step a) is 2 to 20 h, preferably 5 to 12 h, more preferably 7 to 10 h and/or
VI) the initial pressure in step a) is 1 to 20 bar, preferably 1 to 10 bar, more preferably 1 to 5 bar, most preferably 1 to 1.5 bar and/or
VII) the gas flow in step a) is 0.02 to 0.2 vvm, more preferably 0.05 to 0.16 vvm, most preferably 0.1 vvm and/or
VIII) the temperature in step a) is 20 to 40 °C, preferably 25 to 38 °C, more preferably 30 to 39 °C, most preferably 35 to 38 °C and/or
IX) in step c) carbon monoxide and carbon dioxide are present and the ratio of carbon dioxide to carbon dioxide, based on volume-% is 9 to 0.1, preferably 4 to 0.6, more preferably 2.5 to 0.8, most preferably 1.5 and/or
X) the second reactor (R2) is a stirred tank reactor and/or
XI) a reduction agent is present in step c) and/or
XII) a reduction agent is present in step c), selected from the group consisting of cysteine or a salt thereof.

4. The process according to any one of claims 1 to 3, wherein the residence time in step c) is 2 to 20 h, preferably 5 to 15 h, more preferably 10 to 13 h.

5. The process according to any one of claims 1 to 4, wherein the initial pressure in step c) is 1 to 20 bar, preferably 1 to 10 bar, more preferably 1 to 5 bar, most preferably 1 to 1.5 bar.

6. The process according to any one of claims 1 to 5, wherein the pH value is maintained during step c) at a value of about 5.

7. The process according to any one of claims 1 to 6, wherein the gas flow in step c) is 0.01 to 0.1 vvm, preferably 0.03 to 0.08 vvm , more preferably 0.05 vvm

8. The process according to any one of claims 1 to7, wherein the temperature in step c) is 20 to 40 °C, preferably 25 to 38 °C, more preferably 30 to 39 °C and most preferably 35 to 38 °C.

9. The process according to any one of claims 1 to 8, wherein the amount of organic acids or the corresponding salts thereof in the medium of step b), based on the overall weight, is higher than in the medium after finishing step c).

10. The process according to any one of claims 1 to 9, wherein the specific stirring power (P/V) in step a) is 2 to 20 W/L, preferably 6 to 15 W/L, more preferably 8 to 13 W/L.

11. The process according to any one of claims 1 to 10, wherein the specific stirring power (P/V) in step c) is 2 to 20 W/L, preferably 6 to 18 W/L, more preferably 10 to 16 W/L.

12. The process according to any one of claims 1 to 11, wherein the at least one alcohol formed in step a) is an alcohol comprising 1 to 10 carbon atoms, preferably selected from ethanol, butanol and/or hexanol.

13. The process according to any one of claims 1 to 12, wherein the at least one alcohol formed in step c) is an alcohol comprising 1 to 10 carbon atoms, preferably selected from ethanol, butanol and/or hexanol.

14. The process according to any one of claims 1 to 13, wherein in step c) at least one of the gases carbon dioxide or carbon monoxide is present.

15. The process according to any of claims 1 to 14, wherein the dilution rate in step a) is 0.04 to 0.16 h⁻¹and in step c) 0.01 to 0.15 h⁻¹.
